(19) **Europäisches Patentamt European Patent Office Office européen des brevets**

(11) **EP 4 772 114 A1**

(12) **EUROPEAN PATENT APPLICATION**

(43) Date of publication:
**08.07.2026 Bulletin 2026/28**

(21) Application number: **25150034.4**

(22) Date of filing: **02.01.2025**

(51) International Patent Classification (IPC):
**A61B 6/46** (2024.01)   **H01H 3/14** (2006.01)
**H01H 21/26** (2006.01)

(52) Cooperative Patent Classification (CPC):
**A61B 6/467; H01H 3/14; H01H 21/26**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AE Eindhoven (NL)**

(72) Inventors:
- **HOOGEVEEN, Edwin Arnaud**
  **Eindhoven (NL)**
- **CUPPEN, Martinus Antonius Maria**
  **Eindhoven (NL)**
- **DUINEVELD, Paulus Cornelis**
  **5656AG Eindhoven (NL)**
- **EMMEN, Gerlach Corne Pieter Maria**
  **Eindhoven (NL)**

(74) Representative: **Philips Intellectual Property & Standards**
**High Tech Campus 34**
**5656 AE Eindhoven (NL)**

(54) **APPARATUS FOR CONTROLLING X-RAY IMAGING**

(57)     The present invention relates to an X-ray system and an apparatus for controlling X-ray imaging, in particular a footswitch having at least one pedal comprising a pedal plate configured to be moveable by a user operation, and a base plate configured to provide an end position for a movement of the pedal in which contact between the pedal plate and the base plate in established in a contact area having a relatively limited size of 200 mm2 or less. In one aspect, at least one of the base plate and the pedal plate is provided with a cut-out overlapping with at least part of the other one of the based plate and the pedal plate, the contact area being adjacent to the cut-out arranged along a periphery of the cut-out. In another aspect, the contact area is defined by one or more contact structures or elements on a contact side of the base plate and/or the pedal plate.

FIG. 1

**Description**

FIELD OF THE INVENTION

The invention relates to an apparatus for controlling X-ray imaging, in particular a footswitch, and to an X-ray imaging system including such apparatus.

BACKGROUND OF THE INVENTION

In medical X-ray imaging systems, a footswitch having one or more pedals is used to control various functions of the machine, allowing the operator to keep their hands free for other tasks. Typically, one of the functions of the footswitch is to control the activation of the emission of X-rays towards an X-ray detector with which X-ray images of a patient may be captured.

For example, in a footswitch that has been in use with Philips Azurion interventional X-ray systems, the user uses his foot to press down a foot pedal. A metal pedal plate can be pressed until its front edge contacts a metal base plate. When the pedal is pressed down to the end position, a rear section of the plate contacts and activates an electronic switch inside the housing of the footswitch, the switch controlling the emission of X-rays and the generating of X-ray images.

When the user removes his foot and releases the pedal, X-ray emission must be stopped as quickly as possible. For example, there is a need to deactivate the X-ray source relatively quickly when a user releases the pedal, typically within a 50 ms threshold. However, in practice, increased deactivation times are occasionally observed, which might lead to unnecessary X-ray radiation to be received by the patient.

SUMMARY OF THE INVENTION

It would be advantageous to eliminate the occurrence of increased deactivation times of the X-ray source.

To address this, in a first aspect an apparatus for controlling X-ray imaging is provided as defined in the independent claim 1. In a preferred embodiment, the apparatus is configured as a footswitch, wherein the pedal is operable by a foot of the user.

Accordingly, an apparatus for controlling X-ray imaging is provided with at least one pedal having a pedal plate configured to be moveable by a user operation, and a base plate configured to provide an end position for a movement of the pedal plate in which contact between the pedal plate and the base plate in established in a contact area.

At least one of the base plate and the pedal plate is provided with a cut-out overlapping with at least part of the other one of the based plate and the pedal plate, the contact area being arranged along a periphery of the cut-out. In this respect, "overlapping" indicates for example that when the cut-out is provided in the base plate, the cut-out is positioned behind (or in front of) at least part of the pedal plate, as seen from the top (respectively the bottom) of the apparatus. In general, an effect of an overlapping cut-out is that a size of the contact area is reduced.

The present inventors had the insight that a cause of increased deactivation times may be an accumulation of substances, such as contrast agent or blood, between the base plate and the pedal. Spilling of such liquids, for example onto a footswitch, may occur during medical procedures or interventions in which the X-ray system is used.

In particular, for contrast enhanced X-ray imaging (angiography), iodine based contrast agents may be used. Where such contrast agent, or blood from a patient, has spilled on the foot pedal and such contamination is not sufficiently cleaned, the residue may become very viscous and/or obtain a high yield stress due to evaporation of water from the deposited substances.

As a result, when using the X-ray system, over time residue might build up between the plates of the footswitch, to a point where a yield stress of solidified residue counteracts the release of the pedal from the base plate when the user removes his foot from the pedal. In particular in case of insufficient cleaning of the footswitch, the release time of the pedal might exceed 50 ms.

In the conventional design, the contact area would be a relatively large line contact being defined along the width of the front edge of the pedal. It was found that, in this case, a force counteracting pedal release, as exerted by deposited residue in the contact area, would likewise be relatively large, slowing down a release of the pedal.

Therefore, in accordance with preferred embodiments, a cut-out reduces a surface area of the pedal being in contact with the base plate in the end position and thereby a surface area of any solidified residue counteracting the release of the pedal plate. In effect, release of the pedal is thereby facilitated.

In this respect, it is noted that "contact" herein not necessarily involves a direct (e.g. metal-to-metal) contact of the two plates, but that contact between the plates may also be established in use through a thin layer of deposited liquid, whether solidified or not. Thus, typically, a "contact area" may be regarded as an area where a minimum distance between a point on the top plate and a closest point on the bottom plate is smaller than a threshold, for example 0,5 mm. In accordance with the examples described herein, in this case the contact area may then have a size of 200 mm2 or less.

In addition, providing the cut-out for example in the base plate may aid in draining deposited liquids such as blood or contrast agent from the footswitch already before such material solidifies, advantageously reducing build-up of residue and facilitating cleaning of the footswitch.

In an example, the cut-out may be provided as a recess in the front edge of the base plate, wherein the "front edge" shall be understood as the edge of the base plate of the footswitch facing a user when operating the apparatus. The contact area may then extend along an inner edge of such recess, for example, the inner edge being understood as the rear edge of the recess opposing the (open) front of the recess being located in line with the front edge of the base plate.

In certain examples, the base plate and/or the pedal plate comprises at least one protrusion extending into the cut-out, the at least one protrusion defining the contact area. Such protrusions may be provided along the inner edge of the cut-out. For example, protrusions are provided along the rear edge of the cut-out, extending towards the front edge of the base plate.

In certain examples, two such protrusions are provided for each pedal, for instance providing a total contact area of less than 200 mm2, more preferably about 60 mm2.

In other examples, a (further) reduced contact area is provided by means of one or more contact structures or elements on a contact side of the base plate and/or the pedal

For example, one or more contact structures are provided on either plate, the contacts having a spherical shape. While in certain examples, the contact structures may be advantageously combined with a cut-out for further optimizing shape and size of the contact area, alternatively, a reduced contact area may also be provided by means of the contact structures only, without relying on a cut-out in one of the plates. Accordingly, in a further aspect, an apparatus for controlling X-ray imaging is provided as defined in the independent claim 10.

In certain examples, the contact structures provide a total contact area having a size of less than 200 mm2.

In certain examples, the top plate is arranged at an angle with respect to the base plate. This angle $\beta$ is at least 0,75 degrees, for example 0,86 degrees (0,015 radian), although the angle may also be larger than 1 degree or in some examples up to 5 degrees or even more.

In further examples, the base plate is arranged at an angle to a horizontal plane, for example 1 degree or more. Thus, advantageously, an automatic draining of contamination by blood or contrast agent from the apparatus is further facilitated, thereby reducing build-up of residue. For a footswitch, in general, the horizontal plane coincides with the floor of the (hospital) room in which the X-ray equipment with the footswitch is installed or used.

In a further aspect, an X-ray imaging system is provided, comprising an apparatus according to one of the examples described herein, an X-ray detector and an X-ray source configured to emit X-ray radiation when contact is established between the base plate and the pedal plate of the apparatus. In a preferred embodiment, the apparatus is provided as a footswitch, whereby a foot of the user may control the (de)activation of X-ray radiation by pressing and releasing the pedal.

These and other aspects of the present invention will become apparent from and be elucidated with reference to the embodiments described hereinafter.

BRIEF DESCRIPTION OF THE DRAWINGS

Exemplary embodiments of the invention will now be described with reference to the following drawings, which, unless stated otherwise, are schematic and not to scale, wherein:

Fig. 1 shows a top view of an exemplary footswitch;
Fig. 2 shows sectional views of the footswitch of Fig. 1;
Fig. 3 shows sectional views of an alternative exemplary footswitch;
Fig. 4 shows an example of X-ray imaging system with such footswitch.

DETAILED DESCRIPTION OF EMBODIMENTS

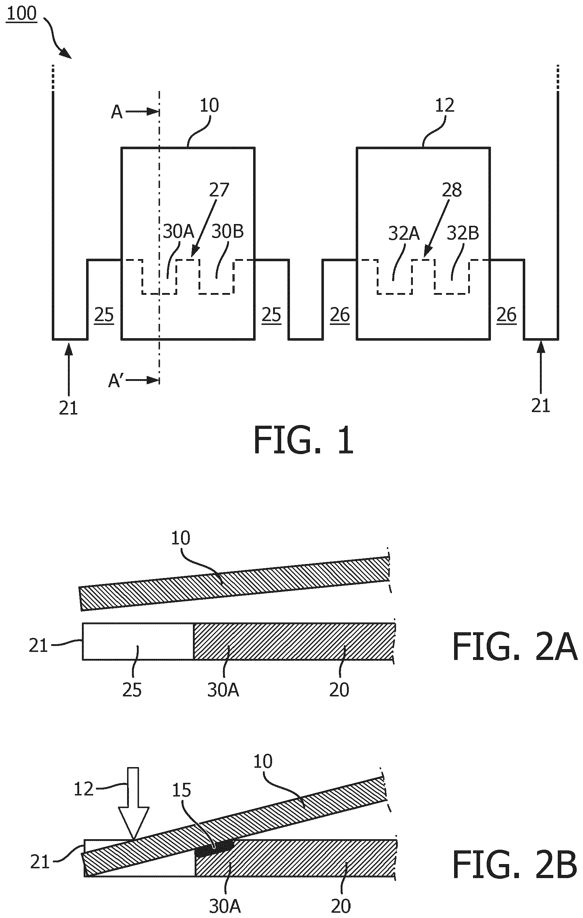

An embodiment of an apparatus for controlling X-ray imaging in accordance herewith is depicted in Fig. 1 showing a top view of a footswitch 100. In this example, a footswitch having two pedals 10, 12 is depicted, however footswitches with three or more pedals, or a single pedal, are readily envisaged herein. In general, individual pedals control different functions of an X-ray imaging system.

At least one of the pedals 10, 12 controls the activation and deactivation of X-ray radiation for acquiring X-ray images. For example, the first pedal 10 may activate a lower dosage fluoroscopy acquisition, while the second pedal 12 may activate a higher dosage X-ray exposure, for example for acquiring contrast enhanced angiographic X-ray images. In this case, a contrast agent is injected into a patient's vasculature so as to increase a visibility of the blood vessels in the X-ray images being acquired. Although the contrast agent is injected, spilling of such liquids and thereby contaminating the footswitch 100 for example is not uncommon during prolonged medical procedures such as X-ray image guided interventions.

The footswitch 100 has a base plate 20, a front edge 21 of which is provided with two recesses defining cut-outs 25, 26 overlapping with the pedals 10, 12. As drawn, the cut-outs 25, 26 extend along and beyond the width of each pedal 10, 12.

When a pedal 10, 12 is pressed down by a foot of a user, the pedal plate contacts the base plate 20 in a contact area substantially along the inner (rear) edges 27, 28 of the cut-outs 25, 26 (dashed lines in Fig. 1). In particular, for each cut-out 25, 26, the inner edge 27, 28 is provided with two protrusions 30A-B, 32A-B defining the contact area 15 between the base plate 20 and each of the respective pedals 10, 12. Thus, a contact area 15 having a relatively limited size is defined in the form of "point contacts" between the pedals 10, 12 and base plate 20. While the protrusions are depicted here as having a square shape, other shapes such as rectangular or tapered shapes are also envisaged herein.

While the protrusions 30A, 30B, 32A, 32B in this example are flat protrusions extending essentially in the plane of the base plate 20, this is not necessarily the case; for example, in embodiments wherein the pedal plates are arranged at a small angle to the base plate, the top surface of the protrusions may be angled similarly, as is depicted in Fig. 2A by way of example.

In a practical implementation, for example, the base plate 20 has a width (horizontal direction in Fig. 1) of 375 mm and is provided with two recesses of 130 mm width extending from the front edge 21 over a depth (vertical direction in Fig. 1) of 30 mm. The recesses correspond to the cut-outs 25, 26. Protrusions 30A, 30B, 32A, 32B having a width of 6 mm and depth of 5 mm extend from the rear edges 27, 28 of cut-outs 25, 26 towards the front side of the base plate 20. In this case, the total contact area between each of the pedals 10, 12 and the base plate 20 corresponds to 60 mm2.

Figs. 2A and 2B show sectional views of the pedal 10 and base plate 20 along the line A-A' depicted in Fig. 1. In Fig. 2A, the pedal 10 is shown in a rest state or unloaded state, in which no contact is made between the pedal plate 10 and the base plate 20 and thus no X-ray images are acquired. In this state, a gap between the base plate 20 and the top plate 10 is preferably at least 10 mm, facilitating the cleaning or removal of contaminating blood or contrast agent from the footswitch 100 when the footswitch is not in use.

In this example, the pedal 10 is arranged at an angle to the base plate 20 and the horizontal plane (e.g. a floor) of about 0,86 degrees (0,015 radian). By providing an angle between the pedal 10 and the base plate 20, at contact a relatively large space remains present between the two at or in the vicinity of contact area 15. For example, at a rear edge 27, 28 of the cut-outs 25, 26, a distance of about 0,45 mm is present between pedal and base plate. As a result, a relatively large volume of liquid can be accommodated at the contact area 15 before, in the loaded state, the negative effects of the residue may become noticeable.

Fig. 2B shows the loaded state of the pedal 10, in which a foot of the user (not shown) presses down on a front portion of the pedal as indicated by arrow 12 to activate X-ray imaging. Here, contact is made between the pedal 10 and the base plate 20 in a contact area 15 essentially corresponding to the protrusion 30A. In particular, it is shown schematically that contact is established through a thin layer of residue. By virtue of the limited surface area of protrusion 30A, the yield stress of the deposited residue in the contact area 15 is relatively low so that a delayed release of the pedal is avoided.

In an alternative embodiment, a contact side 13 of the pedal plate 10 and/or a contact side 23 of the base plate 20 may be provided with contact structures or elements 16, 26 that define similar point contacts. That is, on a side of a plate (e.g. the pedal) facing the other plate (e.g. the base plate), raised structures or elements are provided that define an essentially point-shaped contact area when a user presses down the pedal. Such (point) contact structures may comprise a spherical shape for example.

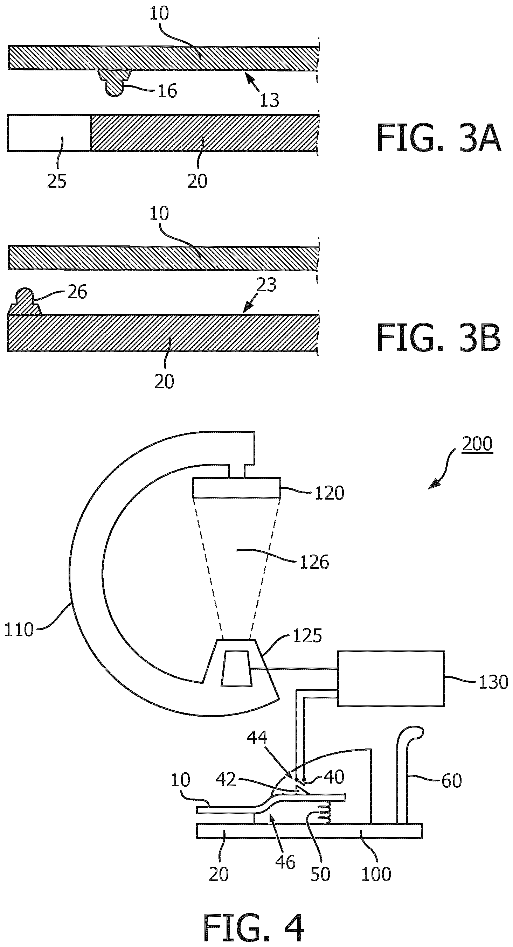

In the example of Fig. 3A a contact structure 16 on the pedal 10 is used in combination with a cut-out 25 in the base plate 20. The contact structure 16 may be arranged such that, in the loaded state, a contact area is provided adjacent an (inner) edge of the cut-out 25.

However, examples wherein only a contact structure is provided, without any cut-out in the base plate or pedal, are also envisaged herein. This is shown in Fig. 3B by way of an example, wherein the base plate 20 is provided with a contact structure 26 adjacent its front edge 21, but no cut-out is provided in either plate. Accordingly, contact between the plates is established substantially at the contact structures 16, 26 advantageously defining a contact area of relatively limited size. Also, in certain examples, contact structures 16, 26 may be provided both on the pedal 10 and the base plate 20, preferably being arranged so that a load caused by the user's foot pressing down on the pedal 10 is optimally distributed.

In certain circumstances, contact structures are described and shown herein may further reduce a release time for the pedal if deposited residue were still present, as compared to a flat strip-shaped protrusion for example. For example, contact structures may be provided having a spherical shape, or comprising a spherical contact element on top of a tapered structure as shown in Fig. 3. In certain examples, a typical sphere diameter could be of the order of between 1 and 10 mm and preferably between 2 to 6 mm, to balance between strength of the construction and short release times. In certain examples, the spherical contact element may comprise a half-sphere having such diameter provided on top of a rod or cylinder for example having a height of at least half of the sphere diameter. Thus, for example, a contact structure with a total height (Z) of at least 2 mm, more preferably at least 5 mm, is provided.

In general, it has been found to be beneficial to have a contact area between the pedal plate and base plate that is as small as possible, as smaller contact areas reduce a surface area where deposited residue may exert a force counteracting the release of the foot pedal as described herein. In particular, the minimum force required to pull the plates apart can be given as:

$$F_{min} \approx \tau_{yield} \cdot A_{contact}$$

where $\tau_{yield}$ is the yield stress of the material and $A_{contact}$ the surface area where the deposited residue e.g. contrast agent is in contact with both the upper and lower plate.

However, on the other hand, the contact area must still be large enough so as not to compromise the strength of construction of the plates. For example, a too small contact area might result in deformations in one or both plates due to the force exerted by the user's foot in operating the pedals.

A typical yield stress of largely solidified contrast agent (e.g. solid mass fraction of 96%) has been found to be about $10^4$ Pa. In addition, as shown in Fig. 3, a spring 50 may be used aiding in the release of the pedal 10, thus counteracting $F_{min}$; the spring 50 typically has a spring force between 10N and 20N. Accordingly, sufficient force is provided to reliably overcome $F_{mm}$ and thus enable easy release of the pedal 10. On the other hand, the spring force is still low enough to not cause too much resistance in operating the pedal in particular over prolonged time periods during X-ray guided interventions for example.

For an exemplary spring force of 17 N being applied, the total contact area should be less than 600 mm2, preferably less than 200 mm2. When the contact area is sized accordingly, the problem of the top plate and base plate sticking together due to deposited residue (solidified contrast agent) is strongly reduced.

As described above, in certain examples, for each pedal, two protrusions may be provided each having a size of about 5 by 6 millimeters, defining the total contact area of about 60 mm2. In experiments, when the contact area was shaped and sized accordingly, sticking together of the pedal and the base plate was no longer observed and the release time consistently met the required 50 ms threshold.

Fig. 4 shown a schematic view of an X-ray imaging system 200 in which a C-arm 110 is provided as a holding arrangement for an X-ray detector 120 and X-ray source 125. However, other holding arrangements such as robotic arms are readily envisaged herein.

During an X-ray guided intervention for example, the C-arm 110 may be positioned around a patient lying on a patient support or table (not shown) so that an X-ray beam 126 emitted from the source 125 to the detector 120 passes through the patient. As different types of material e.g. soft tissues, bones and air cause different levels of X-ray absorption, X-ray images thus captured by the detector 120 show a representation of the patient's inner body structures. Advantageously, during image guided interventions, metal interventional objects and devices, such as guidewires, catheters and stents, are particularly well visible, so that a physician can rely on these X-ray images to guide such devices through a patient's vasculature for example.

In such procedures, it is important that the physician may keep his full attention to a display showing the X-ray images, as he may need to manipulate a device inside the vasculature relying on these images. Thus, in order to control the generating of the X-ray images at minimal distraction to a user, typically a footswitch is used so that the user can control the (de)activation of X-ray imaging by operating a pedal with his foot while keeping his eyes directed at the display.

In Fig. 4, an X-ray imaging system 200 having a footswitch 100 is schematically shown in side view.

In particular, the footswitch 100 comprises at least one pedal 10 and a base plate 20 according to one of the examples described herein. The footswitch 100 further comprises a housing 44, which comprises a rear portion of the pedal 10 that is in connection with an electronic switch 40 and a release spring 50.

When the pedal 10 is pressed down sufficiently far by a user, the electronic switch 40 is activated. In an example, the pedal 10 may comprise two portions being pivotably arranged inside the housing (indicated schematically by pivot point 46) so that a rear or inner portion of the pedal inside the housing 44 may pivot up when the front or outer portion of the pedal 10 is pressed down by a user's foot. Thus, a contact portion 42 of the pedal 10 is brought into contact with an electronic switch 40, activating the switch and thereby providing a control signal, such as a switch-on trigger signal, to control unit 130 for activating the X-ray source 125.

To aid in operating the pedal, a spring 50 may be provided in contact with the pedal 10, whereby the spring is arranged so that the force of the spring 50 provides some resistance in pressing down the pedal and in particular aids in quickly releasing the pedal plate and removing it from contact with the base plate 20, countering any sticking force still exerted by deposited residue.

Thus, when the foot is removed from the pedal, the spring force of spring 50 enables a quick deactivation of the electronic switch 40 within the 50ms threshold, thereby providing a control signal, such as a switch-off trigger signal, to control unit 130 for deactivating the X-ray source and switching off X-ray radiation.

The footswitch 100 further comprises, at a rear edge of the base plate 20, a grip bar 60 that allows a user to pick up and carry the footswitch 100 if this needs to be repositioned within the room. The grip bar 60 for example has a U-shape for easy handling of the footswitch.

In summary, the present invention relates to an X-ray system and an apparatus for controlling X-ray imaging, in particular a footswitch having at least one pedal comprising a pedal plate configured to be moveable by a user operation, and a base plate configured to provide an end position for a movement of the pedal in which contact between the pedal plate and the base plate in established in a contact area having a relatively limited size of 200 mm2 or less. In one aspect, at least one of the base plate and the pedal plate is provided with a cut-out overlapping with at least part of the other one of the based plate and the pedal plate, the contact area being adjacent to the cut-out arranged along a periphery of the cut-out. In another aspect, the contact area is defined by one or more contact structures or elements on a contact side of the base plate and/or the pedal plate.

While the invention has been illustrated and described in detail in the drawings and foregoing description, such illustration and description are to be considered exemplary and not restrictive. The invention is not limited to the disclosed embodiments and examples. Other variations to the disclosed embodiments and examples can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1.  An apparatus (100) for controlling X-ray imaging, comprising:

    at least one pedal (10, 12) comprising a pedal plate configured to be moveable by a user operation, and
    a base plate (20) configured to provide an end position for a movement of the pedal in which contact between the pedal plate and the base plate in established in a contact area (15), wherein at least one of the base plate and the pedal plate is provided with a cut-out (25, 26) overlapping with at least part of the other one of the based plate and the pedal plate, the contact area (15) being arranged along a periphery of the cut-out (25, 26).

2.  The apparatus of claim 1, wherein the cut-out (25, 26) is arranged as a recess in a front edge (21) of the base plate (20).

3.  The apparatus of any preceding claim, wherein the base plate and/or the pedal plate comprises at least one protrusion (30A, 30B, 32A, 32B) extending into the cut-out, the at least one protrusion defining the contact area.

4.  The apparatus of claim 3, wherein two protrusions are provided along an inner edge (27, 28) of the cut-out in the base plate.

5.  The apparatus of claim 4, wherein the two protrusions provide a total contact area of about 60 mm2.

6.  The apparatus of any preceding claim, wherein the contact area is defined by one or more contact structures or elements (16, 26) on a contact side (13, 23) of the base plate and/or the pedal.

7.  The apparatus of claim 6, wherein the one or more contact structures have a spherical shape.

8.  The apparatus of any preceding claim, wherein the pedal plate is arranged at an angle with respect to the base plate of at least 0,75 degree.

9.  The apparatus of any preceding claim, wherein the base plate is arranged at an angle of at least 5 degrees to a horizontal plane.

10. An apparatus for controlling X-ray imaging, comprising:

    at least one pedal (10, 12) having a pedal plate configured to be moveable by a user operation, and
    a base plate (20) configured to provide an end position for a movement of the pedal in which contact between the pedal plate and the base plate in established in a contact area, wherein the contact area is defined by one or more contact structures or elements (16, 26) on a contact side (13, 23) of the base plate (20) and/or the pedal plate (10).

11. The apparatus of claim 10, wherein the contact structures have a spherical shape.

12. The apparatus of any preceding claim, wherein the contact area has a size of less than 200 mm2.

13. The apparatus of any preceding claim, further comprising a switch (40) configured to provide an activation signal for activating the X-ray imaging when the contact is established.

14. The apparatus of any preceding claim, wherein the apparatus is configured as a footswitch and wherein the pedal is operable by a foot of the user.

15. An X-ray imaging system (200), comprising:

    an apparatus (100) according to any preceding claim,
    an X-ray detector (120), and
    an X-ray source (125) configured to emit X-ray radiation when the contact is established.

FIG. 1

FIG. 2A

FIG. 2B

FIG. 3A

FIG. 3B

FIG. 4

| | Europäisches Patentamt European Patent Office Office européen des brevets | **EUROPEAN SEARCH REPORT** | **Application Number** EP 25 15 0034 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 107 970 073 A (GEN ELECTRIC) 1 May 2018 (2018-05-01) | 1,3,4,6, 8-10, 13-15 | INV. A61B6/46 H01H3/14 |
| Y | * (38); | 7 | H01H21/26 |
| A | figures 2,1 * | 2,5,12 | |
| | ----- | | |
| X | US 2020/008896 A1 (CONE TAYLOR JOSEPH [US] ET AL) 9 January 2020 (2020-01-09) | 1,3,4,6, 8-10, 13-15 | |
| A | * (440); figures 4c,4b * | 2,5,12 | |
| | ----- | | |
| X | US 2020/305815 A1 (STEPINA ELIZAVETA [DE] ET AL) 1 October 2020 (2020-10-01) | 10,11, 13-15 | |
| Y | * (46); | 7 | |
| A | figure 3m * | 2,5,12 | |
| | ----- | | |
| X | US 2020/125131 A1 (CONE TAYLOR [US] ET AL) 23 April 2020 (2020-04-23) | 10 | |
| A | * figure 7 * | 2,5,12 | |
| | ----- | | |

| | | | TECHNICAL FIELDS SEARCHED (IPC) |
|---|---|---|---|
| | | | A61B H01H |

The present search report has been drawn up for all claims

| Place of search | Date of completion of the search | Examiner |
|---|---|---|
| Munich | 10 June 2025 | Anscombe, Marcel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.........................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 15 0034

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2025

| Patent document cited in search report | | Publication date | Patent family member(s) | | Publication date |
|---|---|---|---|---|---|
| CN 107970073 | A | 01-05-2018 | CN | 107970073 A | 01-05-2018 |
| | | | EP | 3315080 A1 | 02-05-2018 |
| | | | US | 2018113983 A1 | 26-04-2018 |
| US 2020008896 | A1 | 09-01-2020 | US | 2018280099 A1 | 04-10-2018 |
| | | | US | 2020008896 A1 | 09-01-2020 |
| US 2020305815 | A1 | 01-10-2020 | CN | 111743558 A | 09-10-2020 |
| | | | DE | 102019204287 A1 | 01-10-2020 |
| | | | US | 2020305815 A1 | 01-10-2020 |
| US 2020125131 | A1 | 23-04-2020 | US | 10503199 B1 | 10-12-2019 |
| | | | US | 2020125131 A1 | 23-04-2020 |
| | | | WO | 2020018124 A1 | 23-01-2020 |

EPO FORM P0459

For more details about this annex : see Official Journal of the European Patent Office, No. 12/82